# EUROPEAN PATENT APPLICATION

(11) **EP 2 380 914 A1**
(43) Date of publication of application: **26.10.2011**
(21) Application number: 09831367.9
(22) Date of filing: 07.04.2009
(51) Int. Cl.: C07K 19/00, C07H 21/04, C12N 15/64, C12N 15/70, A61K 38/57, A61P 35/00

(54) **FUSION PROTEINS OF APOPTIN-PROTEIN TRANSDUCTION DOMAIN OF CARBOXYL-TERMINAL END OF EC-SOD**

(30) Priority: 10.12.2008 CN 200810044084
(71) Applicant: Zhejiang Reachall Pharmaceutical Co., Ltd, Zhejiang 322100 (CN)
(72) Inventor: ZHAO, Jian, Dong Yang Zhejiang 322100 (CN); WANG, Fujun, Dong Yang Zhejiang 322100 (CN); FU, Longyun, Dong Yang Zhejiang 322100 (CN); ZHANG, Taozhu, Dong Yang Zhejiang 322100 (CN); SHAN, Hanwen, Dong Yang Zhejiang 322100 (CN)
(74) Representative: Schweiger, Martin
(86) International application number: PCT/CN2009/000379
(87) International publication number: WO 2010/066090

(57) **Abstract**

Provided are fusion proteins of apoptin-protein transduction domain of carboxyl-terminus of EC-SOD, their encoding genes, the recombinant vectors containing said encoding genes, transformants containing said recombinant vectors, as well as the uses and preparation methods thereof. Especially provided are fusion proteins which fuse protein transduction domain of carboxyl-terminus of EC-SOD in the amino acid sequence of SEQ ID NO:1 or its mutants and apoptin in the amino acid sequence of SEQ ID NO:2 or its mutants, said fusion proteins have strong ability of inducing apoptosis of tumor cells, and can be used for manufacturing of medicament for the treatment of tumors.

## Description

### Field of the invention

The present invention relates to biotechnological field. Especially provided are fusion proteins of apoptin-protein transduction domain of carboxyl-terminus of EC-SOD, the encoding genes, the recombinant vectors, the transformants, as well as the uses and preparation methods thereof.

### Background Art

Superoxide dismutases (SOD) are a class of enzymes that catalyses dismutation reaction of superoxide radical anion (O₂^{-.}). SOD of human beings and mammals can be classified into three kinds of isoenzymes, including SOD (Cu, Zn-SOD) in cytosols, SOD(Mn-SOD) in mitochondria and extracellular EC-SOD (also an Cu, Zn-SOD). Among them, the content of EC-SOD [Marklund SL, Bjelle A, Elmqvist LG, et al. Ann Rheum Dis. 1986, 45: 847-851] is found the least in human body [Marklund SL, Bjelle A, Elmqvist LG, et al. Ann Rheum Dis. 1986, 45: 847-851], which mainly exists in extracellular matrix and cell surface of tissues. The amino acid sequence of EC-SOD can be divided into four function domains: a signal peptide with 18 amino acid residues located in N-terminal end; then a domain with 95 residues related to forming of EC-SOD tetramer; a SOD active domain with 98 residues and a protein transduction domain (PTD) with 29 residues enriched with basic amino acids [Hjalmarsson K, Marklund SL, Engstrom A, et al. Proc Natl Acad Sci USA. 1987, 84: 6340-6344]. This protein transduction domain can bind to heparin-like polysaccharide derivatives of cell surface, and then transmembrane transduction of the PTD can be achieved through endocytosis which lead to EC-SOD being located on nucleus [Karlsson K, Marklund SL. Lab Invest. 1989, 60: 659-666].

Apoptin or apoptotic protein derived from the chicken anemia virus is consisted of 121 amino acid residues. Apoptin can induce cell apoptosis which is independent on P53 and insensitive to Bcl-2 in various tumor cells without affecting the normal cells, so it is a promising drug candidate for antitumor selective therapy with great application prospect [Zhuang SM, Shvarts A, van Ormondt H, et.al. Cancer Res. 1995, 55(3): 486-489]. Previous studies have shown that it is necessary for cell apoptosis induced by apoptin that apoptin is transported into cell and then accumulates in nucleus through its nucleus localization sequence, which finally leads to cell apoptosis by some unclear mechanism. Cell apoptosis induced by apoptin is associated with the phosphorylation of its special amino acid residues and the apoptosis is closely related to Caspase-3 pathway [Noteborn MH. Vet Microbiol. 2004, 98(2): 89-94].

Therefore, the key factor for tumor cell apoptosis induced by apoptin is how to rapidly and efficiently transport apoptin into the tumor cell and achieve the transportation of target proteins across the cell membrane. Many of protein transduction domains (PTDs) with protein transportation function have been found, such as TAT domain derived from human HIV virus, antenapedia (ANTP), herpas simplex virus protein VP22, poly arginine sequences, transduction domain of carboxyl-terminus of EC-SOD, *et al*. Generally, PTD comprises a sequence length of less than 20 amino acids, and a highly positive charge domain which is usually formed by a helix structure. PTD can carry various materials including hydrophilic proteins, peptides, DNA and even particulate matters to conduct extra- and inter-cellular transportation thereof, not restricted by cell types [Schwarze SR, Dowdy SF. Trends Pharmaeol Sci. 2000, 21 (2) : 45-48]. However, the efficiency of transportation is closely related to PTD itself and properties of the fused protein thereof. Thus, in order to efficiently transfer a protein across membrane, it is important to select a suitable PTD. To strengthen tumor cell apoptosis induced by apoptin is to find a suitable PTD to rapidly and efficiently transport apoptin into cell, and then exert apoptosis function of apoptin, thereby inhibiting the growth of tumor cell and treating diseases.

### Summary of the invention

To overcome the existing technological problem, the present invention provides an apoptin fusion protein, which can rapidly and efficiently transport apoptin into cell interior and exert apoptosis function, thereby inhibiting tumor cell growth and treating diseases.

Therefore, one aspect of the present invention discloses fusion proteins of apoptin-protein transduction domain of carboxyl-terminus of EC-SOD, wherein the said fusion proteins comprise protein transduction domain of carboxyl-terminus of EC-SOD in the amino acid sequence of, such as SEQ ID NO:1 or its mutants and apoptin in the amino acid sequence of, such as SEQ ID NO:2 or its mutants.

The invention designs to artificially construct fusion proteins by fusing the protein transduction domain of carboxyl-terminus of EC-SOD or its mutant to apoptin or its mutant through genetic engineering. Through the protein transduction domain of carboxyl-terminus of EC-SOD or its mutant, transmembrane transportation of protein can be achieved, which results in the inhibition of tumor cell growth and treatment for disease. Under this design, the present invention includes the said fusion proteins but does not limit to them.

In one embodiment, the said fusion proteins of apoptin-protein transduction domain of carboxyl-terminus of EC-SOD may include following formula: R2-R1, R1-R2, R1-L- R2-L-R1, R1-L-R2, R2-L-R1 or R2-L-R1-L-R1; wherein R1 is an amino acid sequence as described by SEQ ID NO:1 of the said protein transduction domain of carboxyl-terminus of EC-SOD or its mutant, and L is a linkage peptide, R2 is an amino acid sequence as described by SEQ ID NO:2 of the said apoptin or its mutant, but they do not need to be a same therapeutic protein. The linkage peptide includes but does not limit to, such as (GGGGS)_{N}, (GGGS)_{N} or (GGS)_{N}, wherein N are integers greater than one or equal to one, and G represents glycine, S represents serine.

In another embodiment, in the said fusion proteins of apoptin-protein transduction domain of carboxyl-terminus of EC-SOD, the said protein transduction domain of carboxyl-terminus of EC-SOD of SEQ ID NO:1 or its mutant is located at carboxyl-terminus of the said fusion protein.

In one embodiment, the said fusion proteins of apoptin-protein transduction domain of carboxyl-terminus of EC-SOD are proteins represented by following (a) or (b):
(a) protein possessing an amino acid sequence of SEQ ID NO:3;
(b) protein possessing at least 60% sequence homology to the amino acid sequence of (a) and capable to induce cell apoptosis.

One aspect of the present invention discloses a polynucleotide molecule encoding the said fusion protein of apoptin-protein transduction domain of carboxyl-terminus of EC-SOD.

In one embodiment, the amino acid sequence of the said polynucleotide molecules is SEQ ID NO:4.

One aspect of the present invention discloses a recombinant expression vector of the said polynucleotide molecule of the present invention.

One aspect of the invention discloses a transformant of the said recombinant expression vectors of the present invention.

In one embodiment, the said transformant is *E*. *coli.*

Another aspect of the present invention discloses a method for preparing the said fusion proteins of apoptin-protein transduction domain of carboxyl-terminus of EC-SOD, including following steps:
1. Constructing expression vector of the fusion proteins of apoptin-protein transduction domain of carboxyl-terminus of EC-SOD;
2. Preparing transformant of the fusion proteins of apoptin-protein transduction domain of carboxyl-terminus of EC-SOD;
3. Expressing the fusion proteins of apoptin-protein transduction domain of carboxyl-terminus of EC-SOD;
4. Purifying the fusion proteins of apoptin-protein transduction domain of carboxyl-terminus of EC-SOD.

Another aspect of the present invention discloses a composition comprising the said fusion proteins of apoptin-protein transduction domain of carboxyl-terminus of EC-SOD and pharmaceutically acceptable carriers.

Another aspect of the present invention discloses use of the said fusion proteins of apoptin-protein transduction domain of carboxyl-terminus of EC-SOD in preparing drugs for treatment of disease caused by excessive cell proliferation.

In one embodiment, the said disease caused by excessive cell proliferation is tumor.

Another aspect of the present invention includes methods for preventing, treating or improving disease or disorder caused by excessive cell proliferation, which comprises administering to a mammalian in need an effective amount of fusion proteins of apoptin-protein transduction domain of carboxyl-terminus of EC-SOD to prevent, treat or improve disease or disorder.

Tumor cell apoptosis experiments in vitro and test of inhibiting mouse ascites tumor have shown that the fusion protein of apoptin-protein transduction domain of carboxyl-terminus of EC-SOD prepared in the present invention has a strong capability to induce tumor cell apoptosis, and can be used for preparation of antitumor drugs.

### Brief description of the drawings

FIGURE 1 shows 2% agarose gel electrophoresis spectrum of gene fragment of protein transduction domain of carboxyl-terminus of EC-SOD prepared through PCR amplification.
FIGURE 2 is a schematic diagram for constructing expression plasmid C-pET28a.
FIGURE 3 shows 1.5% agarose gel electrophoresis spectrum of apoptin gene fragment prepared through PCR amplification.
FIGURE 4 shows a schematic diagram for constructing clone plasmid Apop-pMD18T.
FIGURE 5 is a schematic diagram for constructing expression plasmid ApopC-pET28a.
FIGURE 6 shows detection of inserted fragment of recombinant bacteria through bacterial PCR by using 1.5% agarose gel electrophoresis spectrum
FIGURE 7 shows analysis result of electrophoretic spectrum of purified recombinant Apoptin-EC-SOD-PTD SDS-PAGE.
FIGURE 8 shows effect of different concentration of recombinant Apoptin-EC-SOD-PTD on survival rate of HeLa cell.
FIGURE 9 shows effect of recombinant Apoptin-EC-SOD-PTD on survival rate of normal human liver cells cultured in vitro.

### Detailed description of the invention

In the present application, the term of "mutant" refers to mutant with an amino acid sequence of protein transduction domain of carboxyl-terminus of EC-SOD as described by SEQ ID NO:1 or amino acid sequence of apoptin as described by SEQ ID NO:2. Compared to natural protein transduction domain of carboxyl-terminus of EC-SOD or apoptin protein, the mutant has higher activity than its wild type species and/or changed stereo specificity. Amino acid sequence mutant of natural protein can be prepared by introducing appropriate nucleotide changes into the nucleotide of the present invention or synthesizing peptide required *in vitro.* These mutants include, for example, deletion, insertion or replacement of amino acid residues. Final protein product can obtained through combination of deletion, insertion or replacement.

Percentage of protein homology can be determined by GAP analysis (GCG program) (Needleman and Wunsh, 1970), wherein parameters are gap creation penalty is 5 and gap extension penalty is 0.3. If analyzed sequences have a length of at least 15 amino acids, GAP analysis will be conducted in domain possessing at least 15 amino acids of the two sequences detected. Preferably, when analyzed sequences have a length of 50 amino acids, GAP analysis will be conducted in domain with at least 50 amino acids of the two detected sequences. Preferably, when analyzed sequences have a length of 100 amino acids, GAP analysis will be conducted in domains with at least 100 amino acids of the two detected sequences. Preferably, when analyzed sequences have a length of 250 amino acids, GAP analysis will be conducted in domains with at least 250 amino acids of the two detected sequences. Even preferably, when analyzed sequences have a length of at least 500 amino acids, GAP analysis will be conducted in domains with at least 500 amino acids of the two detected sequences.

The present invention relates to aspects including fusion proteins of apoptin-protein transduction domain of carboxyl-terminus of EC-SOD analogues, which are obtained by different modification during or after the synthesis, such as, biotinylation, benzylation, glycosylation, acetylation, phosphorylation; derivation of known protected/closed group, cleavage by hydrolysis of protein and binding to antibody molecules or other cell ligands, *et al*. These modifications can increase stability and/or bioactivity of fusion proteins of apoptin-protein transduction domain of carboxyl-terminus of EC-SOD described in this invention.

### Expression of fusion protein

The present invention includes DNA encoding the fusion proteins of apoptin-protein transduction domain of carboxyl-terminus of EC-SOD, the vector and transformant thereof.

In this invention, the term "transformant" refers to host cells carried with a heterologous DNA molecule.

The present invention also includes methods for producing the fusion proteins of apoptin-protein transduction domain of carboxyl-terminus of EC-SOD of the present invention through synthesis or recombination technology. Isolation and purification of polynucleotide (DNA or RNA), vector, transformant and organism can be achieved by known technology of this art.

Vectors used in this invention can be phage, plasmid, cosmid, mini-chromosome, virus or retrovirus vector. Vectors used for clone and/or expression of polynucleotide are capable to clone and/or express polynucleotide in host cells in which the polynucleotide needs to be cloned and/or expressed. Generally, polynucleotide and/or vector can be used in any eukaryotic or prokaryotic cells, including cells of mammalian (such as human (eg, HeLa)), monkey (eg, Cos), rabbit (eg rabbit reticulocyte), rat, hamster (eg, CHO, NSO and baby hamster kidney cell) or mouse cells (eg L cell), plant cells, yeast cells, insect cells or bacterial cells (such as E. coli). Examples of vector suitable for various types of host cells are referred to F. Ausubel et al., Current Protocols in Molecular Biology., Greene Publishing Associates and Wiley-Interscience (1992) and Sambrook *et al*. (1989). Host cells containing these polynucleotides can be used to abundantly express proteins useful for preparation of drugs, diagnostic reagents, vaccines and therapeutic agents.

Various methods have been developed to operatively link polynucleotide to the vectors via complementary sticky ends. For example, complementary sequence fragment of homopolymers is inserted into DNA segment which is designed to be inserted a vector, and then the vector is linked to the DNA segment by hydrogen bond between the complementary homopolymer tails to form the recombinant DNA.

Synthetic linker with one or more restriction sites provides another alternative method for connecting DNA segment and vector. The DNA segment produced through restriction endonuclease digestion is treated with the bacteriophage T4 DNA polymerase or *E. coli* DNA polymerase I, salient γ-single-stranded end is removed by the two said polymerases through the 3',5'-exonuclease activity, and 3'-recessed end is filled through the polymerization activity thereof. Consequently, flat-ended DNA segment is produced through combination of these activities. The flat-ended segment is incubated with excess linker molecule in a large molar in the presence of enzyme catalyzing connection of flat-ended DNA molecular, such as bacteriophage T4 DNA ligase. Therefore, the product is a DNA segment containing a sequence of polymerized joint end. These DNA segments are cleaved by appropriate restriction enzymes and linked to the expression vectors which have already been disintegrated by enzymes, and the said enzymes can generate end compatible with the said DNA segment. The synthesized linker with more restriction endonuclease sites are commercial available from a number of suppliers.

Polynucleotide inserts should be operatively connected to appropriate promoters compatible with host cells expressing polynucleotide. The promoter can be a strong promoter and/or inducible promoter. Examples of promoters include the phage λ P_{L} P_{R} promoter, *E*. *coli* lac, trp, phoA, tac promoter, SV40 promoter at early and later stage, and retrovirus LTR promoter. Other suitable promoters are known to the skilled person in the art. The expression recombinant vectors further contain transcription initiation and termination sites, and ribosome binding site located in transcription domain. The encoding part of transcript expressed by the recombinant vectors may contain start codon located at the beginning and the stop codon (UAA, UGA or UAG) appropriately located at the end of the translated peptide.

As above mentioned, the expression vector may contain at least one selectable marker. The said marker includes dihydrofolate reductase for eukaryotic cells culture media, G418, glutamine synthase or neomycin resistance, as well as the resistance gene against tetracycline, Kanamycin or Ampicillin used for culture of E. coli or other bacteria. Reprehensive examples of appropriate hosts include, but not limited to, bacterial cells such as *E*. *coli,* Streptomyces and Salmonella typhimurium cells; fungal cells such as yeast cells (eg Saccharomyces cerevisiae or Pichia pastoris); insect cells such as drosophila S2 and Spodoptera SF9 cells; animal cells such as CHO , COS , NSO , 293 and Bowes melanoma cells; and plant cells. The appropriate culture media and cultivation conditions for the host cells mentioned above are known in this field.

Tagged proteins or tagged peptides (Tag) that can be readily isolated and purified are generally used in order to effectively isolate and purify or secrete the target protein. Commonly used tagged proteins include glutathione-S-transferase (GST), hexamer of histidine peptide (His, Tag), protein A and cellulose binding domain, *et al*. After expression, the special properties of the said tagged proteins or tagged peptides can be used to isolate and purify target protein through forming fusion protein by fusing the special protein or peptide to the target protein, such as the specific binding between His Tag and Ni-chelating Sepharose. After purification, sequence of the said tagged protein or tagged peptide in the fusion proteins can be removed by digestion using site-specific protease, such as thrombin, enterokinase and factor Xa, and then the target protein can be obtained.

The present invention also includes host cells comprising the nucleotide sequences disclosed in the invention, wherein the said nucleotide sequences are operatively linked to one or more heterologous control domain, such as promoter and/or enhancer by using the known technology of this art. Host strains that can regulate expression of the inserted gene sequences or modify and process gene product in a required manner are selected. In the presence of certain inducers, expression promoted by some promoters is increased, therefore the expression of peptides genetically modified can be controlled. In addition, different host cells have their own characteristic and specific mechanism of protein translation, post-translational processing and modification, such as phosphorylation and cleavage. Appropriate cell lines are selected to ensure a desired modification and processing on the expressed exogenous proteins.

The nucleotide and nucleotide recombinant vectors described in the present invention can be inserted into host cells through calcium phosphate transfection, transfection mediated by DEAE-dextran, cationic lipid-mediate transfection, electroporation, transduction, infection or other methods. The said methods are described in many standard laboratory manuals, such as Basic Methods in Molecular Biology by Davis et al. (1986).

Polynucleotide encoding fusion protein described in this invention can be connected to vectors containing selected marker, and then proliferate in the host. Generally, a plasmid vector can be introduced into a host cell through calcium chloride transformation or liposome transfection. If the vector is a virus, it can be packaged with suitable packaging cell lines in vitro before inserted into host cells.

Successfully transformed cells, namely the cells with DNA recombinant vectors described in this invention, can be identified by well-known technology. For example, desired peptides can be generated by culture cells inserted with expression recombinant vectors. The cells are collected and disintegrated, and the existence of DNA in the DNA contents can be detected through methods such as those described by Southem (1975) J. Mol. Biol. 95, 503 or by Berent et al (1985) Biotech. 3, 208. Alternatively, the existence of protein in the liquid supernatant can be determined by using antibody method.

It is preferred to recover and purify fusion protein of this invention from recombinant cell culture by using well-known technology, wherein the said technology include ammonium sulfate precipitation, ethanol precipitation, acid extraction, anionic or cationic exchange chromatography, phosphate cellulose chromatography, hydrophobic interaction chromatography, affinity chromatography, hydroxyapatite chromatography, hydrophobic charge interaction chromatography and lectin chromatography. In some embodiments, HPLC may be used for purification.

In some embodiments, one or more chromatography methods mentioned above can be used to purify fusion protein of the present invention. In other embodiments, one or more chromatographic columns mentioned as follows can be used to purify fusion protein of this invention, wherein the said chromatographic columns include Q sepharose FF column, SP sepharose FF column, Q sepharose High Performance column, Blue sepharose FF column, Blue column, Phenyl Sepharose FF column, DEAE Sepharose FF, Ni-Chelating Sepharose FF column, Methyl column, and so on.

In addition, methods disclosed in international application No. WO 00/44772 can be used to purify the fusion protein of this invention, the contents in its entirety is herein incorporated by reference. It is easy for technicians of this field to modify the described methods of purification of fusion protein of this invention. The fusion protein of the present invention can be recovered from products generated by recombinant technology from the prokaryotic or eukaryotic host cells, such as bacteria, yeast, higher plant, insect and mammalian cells.

In one preferred embodiment, the present invention provides a method for preparing fusion proteins of apoptin-protein transduction domain of carboxyl-terminus of EC-SOD, which includes following steps:
a. Constructing expression plasmid C-pET28a containing cloned EC-SOD carboxyl-terminal protein transduction domain gene;
b. Constructing cloned plasmid Apop-pMD18T cloned with a apoptin gene.
c. Constructing expression plasmid ApopC-pET28a cloned with gene of fusion proteins of apoptin-protein transduction domain of carboxyl-terminus of EC-SOD.
d. Expressing and purifying the fusion proteins of apoptin-protein transduction domain of carboxyl-terminus of EC-SOD.

Procedures of constructing expression plasmid C-pET28a of (a.) include: to design 5' and 3' terminal primers; and then to clone EC-SOD carboxyl-terminal protein transduction domain (amino acid residues from NO:196 to 222) within EC-SOD-pSK plasmid containing cloned whole EC-SOD gene sequence through PCR. Then N-terminal of the segment is connected to a linkage peptide possessing 8 amino acid residues, and the obtained segment is cloned into Hind III and Xho I endo-restriction enzyme site of pET28a to form expression plasmid C-pET28a of EC-SOD carboxyl-terminal protein transduction domain gene.

Procedures of constructing cloned plasmid Apop-pMD18T mentioned in(b.) include: to design 5' and 3' terminal primers, and then to clone the whole apoptin gene (amino acid residues from NO:1 to 121) within the Apoptin-pVK plasmid containing cloned whole apoptin gene through PCR method. Then 5' and 3' ends of the gene are connected with Nde I and BamH I restriction site, respectively, and the obtained fragment is then cloned into pMD-18T plasmid to form clone plasmid Apop-pMD18T containing cloned apoptin gene.

Procedures of constructing expression plasmid ApopC-pET28a of (c.) include: to isolate target segment from the cloned plasmid Apop-pMD18T; then this segment is inserted into the C-pET28a expression vector treated with double digestion with enzyme Nde I and BamH I to form expression plasmid ApopC-pET28a with cloned gene of fusion proteins of apoptin-protein transduction domain of carboxyl-terminus of EC-SOD.

Procedure of expressing and purifying fusion proteins of apoptin-protein transduction domain of carboxyl-terminus of EC-SOD of (d.) include: to transfer the constructed recombinant plasmid ApopC-pET28a into the *E*. *coli* hosts fused with T7 RNA polymerase gene. The transferred hosts can be *E*. *coli* BL21(DE3), BL21(DE3)pLysS, JM109(DE3), Rosetta(DE3) and Rosetta DE3 pLysS, *et al*. The constructed engineering bacteria is cultured in LB media in the presence of 50µg/ml Kalamycin at 37°C with shaking for 0.3-0.6 hour, and OD is reached to 600nm. Then the culture media is added IPTG till a final concentration of 0.5-3 mM, and induced culture for 3-5 hours. The cells are collected and treated with ultrasonication, and inclusion bodies are collected by centrifugation. After detection by SDS-PAGE electrophoresis, the inclusion bodies are dissolved and denatured in 8M urea solution. The obtained mixture is isolated through nickel-affinity column chromatography, and target protein is fractional collected, then renatured by dilution, and concentrated. After sterile filtration, protein concentration is measured and the protein is kept for later use.

### Application

The fusion protein of the present invention can be used as an active ingredient for treatment of various kinds of diseases caused by excessive cell proliferation, such as tumor, including but not limited to: bone cancers, including: Ewing sarcoma, osteosarcoma, chondrosarcoma, etc.; brain and CNS tumors, including: acoustic neuroma, neuroblastoma, glioma and other brain tumors, spinal cord tumors, breast cancer, colorectal cancer, advanced colorectal cancer; endocrine carcinoma of categories, including: adrenal cortical carcinoma, pancreatic cancer, pituitary cancer, thyroid cancer, parathyroid cancer, breast cancer, multiple endocrine neoplasia; gastrointestinal cancer categories, including: stomach, esophagus, small intestine, liver, extrahepatic bile duct cancer, gastrointestinal carcinoid tumor, gallbladder cancer; genitourinary cancer categories, including: testis cancer, penile cancer, prostate cancer; gynecological cancer categories, including: cervical cancer, ovarian cancer, vaginal cancer, uterine / endometrial cancer, vaginal cancer, gestational trophoblastic tumor, fallopian tube cancer, uterine sarcoma; head and neck cancer categories, including: oral cancer, lip cancer, saliva gland cancer, laryngeal cancer, hypopharyngeal cancer, is pharyngeal cancer, nasal cavity, paranasal sinus cancer, nasopharyngeal carcinoma; leukemia classes, including: childhood leukemia, acute lymphoblastic leukemia, acute myeloid leukemia, chronic lymphocytic leukemia, chronic myelogenous leukemia, hairy cell leukemia, acute promyelocytic leukemia, plasma cell leukemia; bone marrow cancer, blood disorders, including: bone marrow dysplastic syndrome, myeloproliferative disorders, aplastic anemia, Fanconi anemia, idiopathic macroglobulinemia; lung cancer categories, including: small cell lung cancer, non-small cell lung cancer; lymphoma categories, including: Hodge Golden disease, non-Hodgkin's lymphoma, cutaneous T-cell lymphoma, peripheral T-cell lymphocytic tumors, AIDS-related lymphoma; eye cancer categories, including: retinoblastoma, uveal melanoma; skin cancer categories, including: melanoma, non-melanoma skin cancer, Merkel cell carcinoma; soft tissue sarcoma type, such as: children of soft tissue sarcoma, adult soft tissue sarcoma, Kaposi sarcoma; urinary system cancers, including: kidney Virginia Williams cancer, skin cancer, bladder, urethra cancer or metastatic carcinoma.

The fusion protein disclosed in this invention can be used for treatment of cancers, most preferably liver cancer or lung cancer.

The fusion protein disclosed in this invention can be used for treatment of tumors, preferably solid tumors and hematopoietic system malignancies.

The term "cancer or tumor" used in the present application generally refers to diseases generally characterized by abnormal and uncontrolled growth of cells.

Effective amount of the active ingredient can be adjusted depending on administration mode or severity of the diseases been treated. For most large mammals, total daily amount of the active ingredient administrated is about 0.01-1000mg. Generally the amount range of clinical administration for adult is 0.01-200mg/day, preferably 0.05-100 mg/day.

Both "effective amount" and "therapeutic amount" refer to amount sufficient to cause therapeutic effect. Drug with an effective amount can be administered in once or many times. Typically, effective amount refers to the amount sufficient to alleviate, improve, stabilize, slow or delay further development of diseases.

### Composition

"Composition" refers to composition applied to the present invention or containing fusion protein of the present invention. Typically, when the composition of this invention is applied to the purposes mentioned above, the said fusion protein may be combined with one or more pharmaceutically acceptable carriers or excipients, and formulated into different dosage forms for different administration routes, such as tablets, capsules, powder, granules, syrup, solution, oral liquid, spiritus, tincture, aerosol, aerosol powder, injection, sterile powder for injection, suppository and so on.

"Pharmaceutically acceptable" component refers to material which is applied to human and/or animal without excessive adverse side effects, such as toxicity, irritation and allergic reactions; that is, it has a reasonable benefit/risk ratio. "Pharmaceutical acceptable carrier" refers to the pharmaceutically or alimentally acceptable solvent, suspension agent or vehicle used to transport the fusion protein of this invention into animal or human. The carrier can be liquid or solid.

Fusion protein of this invention can be administrated by oral, intravenous, intramuscular or subcutaneous routes.

Oral dosages forms mentioned above include: tablets, capsules, powders, granules, syrup, solution and spiritus. Solid carriers include: starch, lactose, dicalcium phosphate, microcrystalline cellulose, sugar, kaolin, silica powder, talc, lower substituted hydroxypropyl cellulose, carboxymethyl starch sodium and polyvinylpyrrolidone. Liquid carriers include: sterile water, ethanol, polyethylene glycol, non-ionic surfactants and edible oils (such as corn oil, peanut oil and sesame oil.) Adjuvants normally used during the drug composition preparation include: flavoring agents, coloring agents, preservatives (such as butyl hydroxybenzoate, sodium benzoate, sorbic acid) and antioxidants (such as vitamin E, vitamin C, sodium metabisulfite and butylated hydroxy toluene).

Dosage forms for injection mentioned above include injection and sterile power for injection, which can be formulated by mixing pharmaceutical active ingredient with one or more pharmaceutically acceptable carriers to form a composition for injection. Solvents include: sterile water, ethanol, glycerol, propylene glycol and polyethylene glycol. Besides, extra additives include: the antimicrobial agents (such as benzyl alcohol, butyl hydroxybenzoate, merthiolate), isotonic adjustors (such as sodium chloride, glucose), suspending agents (such as sodium carboxymethyl cellulose, methyl cellulose), solubilizers (Tween-80, egg phospholipids), antioxidants (such as vitamin E, vitamin C, sodium metabisulfite) and fillers (such as lactose, mannitol).

Considering for ease of preparing and administering, the preferred pharmaceutical composition is solid form, more preferably is frozen powder injection, and preferable routes for administration is intravenous.

Following embodiments further explain the present invention. It should be understood that these embodiments are only used to illustrate the invention but not to limit the invention scope. The experimental method in following embodiments without specific conditions should be deemed to be under general conditions, or conditions recommended by manufacturer or supplier. Unless otherwise specified, the ratios and percentages are based on weight.

### Example 1. Constructing expression plasmid C-pET28a containing cloned EC-SOD carboxyl-terminal protein transduction domain gene

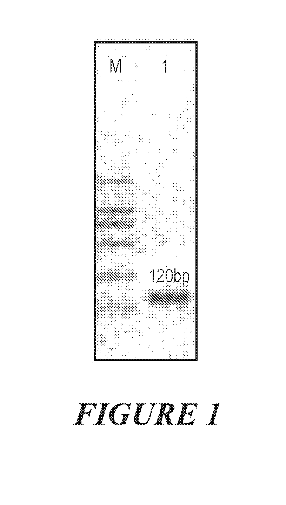

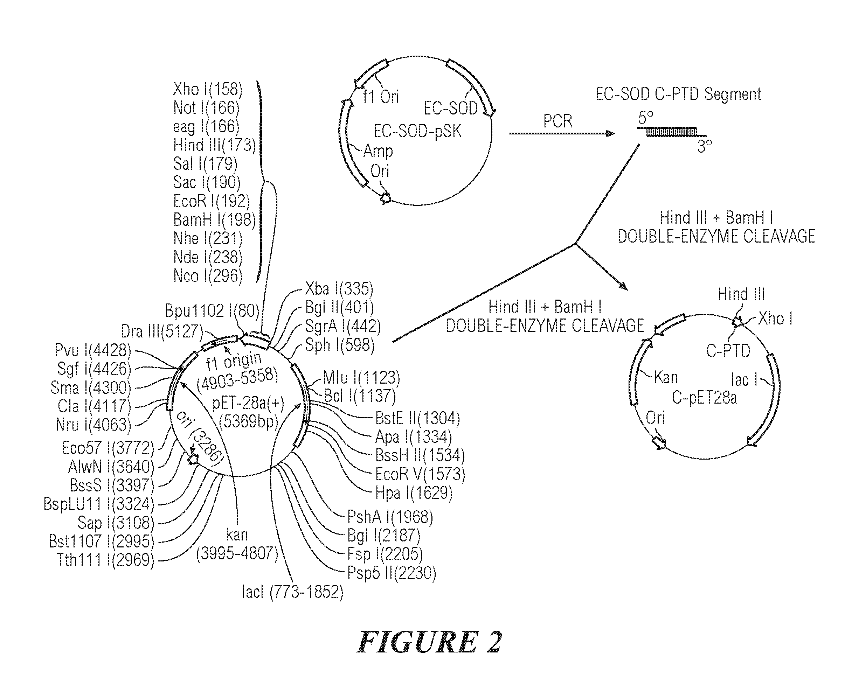

1. PCR Amplification of EC-SOD carboxyl-terminal protein transduction domain gene
   (1) According to the human EC-SOD gene sequence published in Genbank, forward and reverse primer of uncloned EC-SOD carboxyl-terminal protein transduction domain gene were designed.
      P1: 5'- TAAT***AA******GCTT***CCGCTGGAGGCGGTGGAAGCGGGCCCGGGCTC -3'
      P2: 5'- AT***CTCGAG***GGCGGCCTTGCACTCGCTCT -3
      A Hind III restriction site and a linkage peptide with eight amino acid residues (AlaSerAlaGlyGlyGlyGlySer) were introduced into the forward primer P1; whereas, an Xho I restriction site was introduced into the reverse primer P2.
   (2) Procedures of PCR amplification were: to denature at 94°C for 5min; then to denature at 94°Cfor 30s; after that, to renature at 60°Cfor 30s and extend at 72°C for 1min. All these steps were repeated for 30 times and finally it was kept at 72°C for 5min.
   (3) PCR product was recovered and double digested by restriction enzyme Hind III and Xho I, then kept for later use.
2. Constructing recombinant expression plasmid C-pET28a
   The insert fragment obtained by PCR amplification and digestion was connected to the recovered DNA of plasmid pET28a which has been treated with the same enzymatic hydrolysis. The *E*. *coli* DH5α strain was transformed by the connected segment, and then cultured. The recombinant plasmid DNA was recovered and verified by sequencing.
3. 2% Agarose gel electrophoresis spectrum of the target fragment prepared by PCR amplification is shown in FIGURE 1; wherein M represents standard molecular weight of DNA Marker, 1 represents the gene fragment (120bp) of carboxyl-terminal protein transduction domain of EC-SOD prepared by amplification.
4. Schematic diagram for construction procedure of expression plasmid was shown in FIGURE 2

### Example 2: Constructing cloning plasmid Apop-pMD18T inserted with the apoptin gene

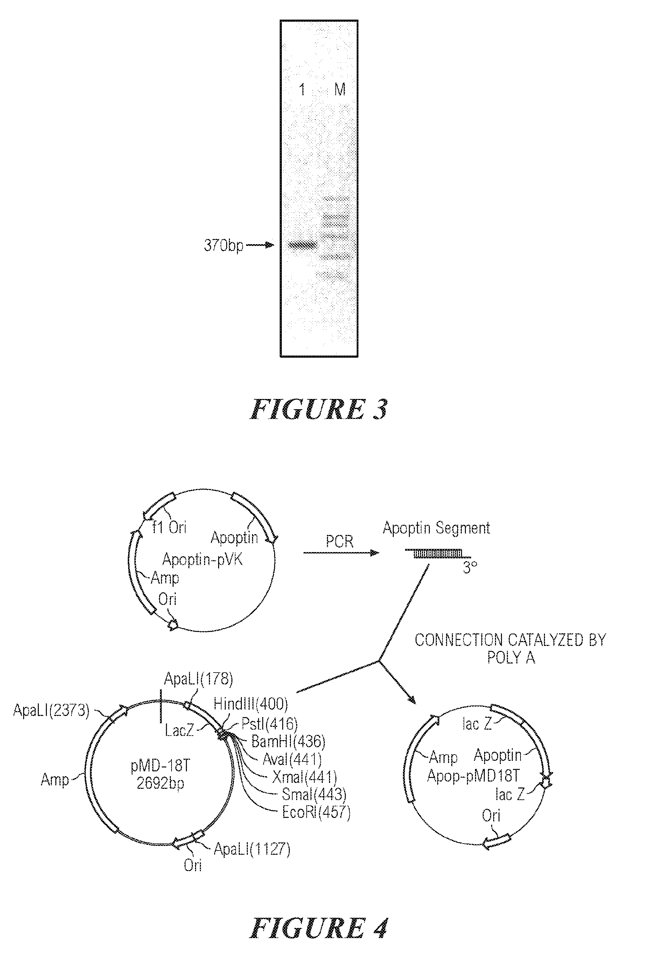

1. PCR Amplification of apoptin gene
   (1) According to gene sequence of chicken anemia virus VP3 protein published in Genbank, forward and reverse primer for whole subcloned apoptin gene were designed.
      P3: 5'- GA***CATATG***ATGAACGCTCTCCAAGAAGA -3'
      P4: 5'- TG***GGATCC***TTACAGTCTTATACGCCTTTTTG -3'
      An Nde I restriction site was inserted into the forward primer P3, and a BamH I restriction site and a stop codon were inserted into the reverse primer P2.
   (2) Procedures of PCR amplification were: to denature at 94°C for 5min; then to denature at 94°Cfor 30s; after that, to renature at 55°Cfor 30s and extend at 72°C for 45s. All these procedures were repeated for 30 times and finally it was kept at 72°C for 5min.
   (3) PCR product was recovered and saved for later use.
2. Constructing recombinant cloning plasmid Apop-pMD18T The insert segment recovered from PCR amplification was connected to DNA of pMD18T vector (purchased from TaKaRa Biotechnology Co.,Ltd.), transformed *E*. *coli* DH5α strain and cultured. Then positive clones were screened by blue-white selection, which were cultured in liquid media. Finally, the recombinant plasmid DNA was recovered and verified by sequencing.
3. 1.5% Agarose gel electrophoresis spectrum of whole apoptin gene segment prepared by PCR amplification is shown in FIGURE 3, wherein 1 represents the whole apoptin gene with about 370bp prepared by PCR, and M represents standard molecular weight of DNA Marker.
4. Schematic diagram for construction of expression plasmid was shown in FIGURE 4.

### Examples 3: Constructing expression plasmid ApopC-pET28a cloned with gene of fusion proteins of apoptin-protein transduction domain of carboxyl-terminus of EC-SOD

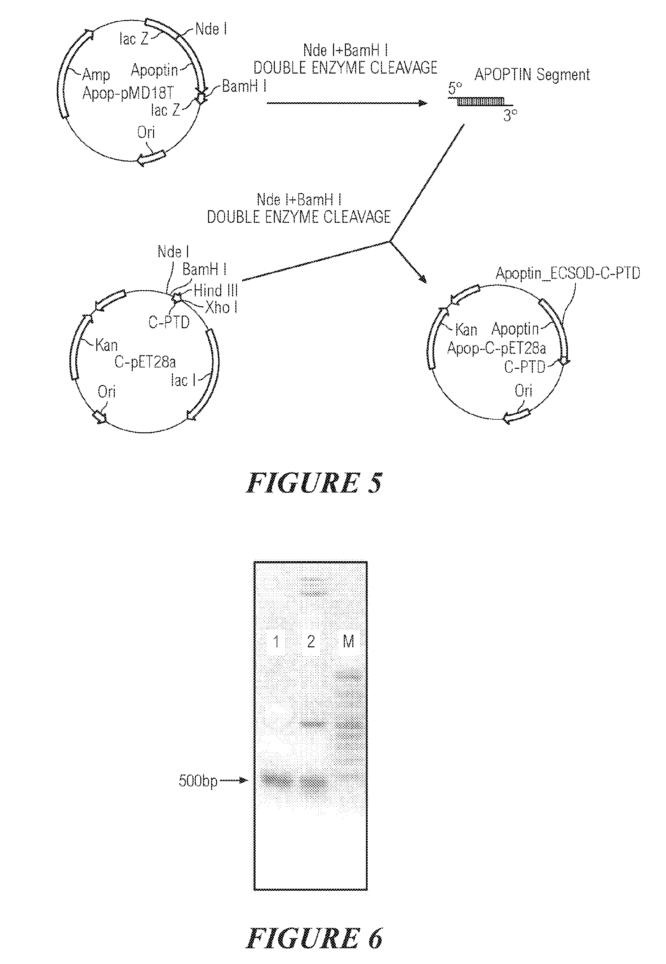

1. Obtaining insert segment of apoptin gene
   DNA of Apop-pMD18T plasmid obtained and verified by sequencing was double digested by Nde I and BamH I enzymes. The digested fragment was isolated through 1% agarose gel electrophoresis and the target DNA segment was recovered by gel extraction mini kit.
2. Preparing vectors
   DNA of C-pET28a plasmid prepared from construction and amplification was also double digested by Nde I and BamH I enzymes. The digested fragment obtained was isolated through 1% agarose gel electrophoresis and the target DNA segment was recovered by gel extraction mini kit.
3. Constructiing recombinant expression plasmid ApopC-pET28a
   The recovered DNA segment of apoptin was linked to DNA of C-pET28a plasmid, and then transformed *E*. *coli* BL21 (DE3) strain, cultured at 37°C. DNA plasmid was then recovered and verified by sequencing.
4. Schematic diagram for construction of expression plasmid ApopC-pET28a is shown in FIGURE 5.
5. 2% Agarose gel electrophoresis spectrum of the recombinant bacteria colony detected through PCR is shown in FIGURE 6, wherein 1 represented negative control using DNA of pET28a plasmid as template; 2 represented PCR detecting result of constructed ApopC-pET28a, wherein primers were P3 and P2, and fragment length was about 500bp; M was standard molecular weight of DNA Marker.

### Example 4. The expression and purification of fusion proteins of apoptin-protein transduction domain of carboxyl-terminus of EC-SOD

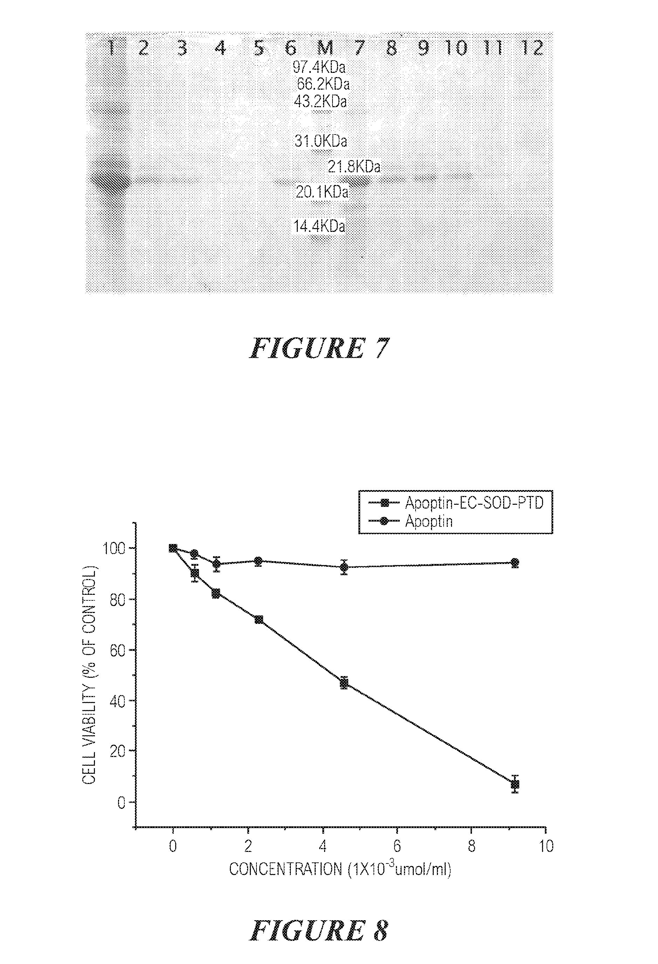

1. The recombinant *E*. *coli* BL21 (DE3) engineering bacteria containing plasmid ApopC-pET28a were cultured on LB agar dishes in presence of Kanamycin. A single clone was selected, cultured in the LB culture media containing 50µg/ml Kanamycin at 37°C overnight. This culture solution was transferred to a new LB media containing 50µg/ml Kanamycine by using 1% inoculum, and then cultured until the its OD600 reaching 0.3-0.6. 0.5-3mM IPTG was added for induction, and the resulted was continued to culture at 37°C for 3-5 hours. The bacterial cells were collected, sonicated, and the inclusion bodies were collected through centrifugation.
2. Bacterial cells obtained from inducible expression were sonicated, and then analyzed by 15% SDS-PAGE electrophoresis (sees FIGURE 7). In FIGURE 7, 1 represents supernatant of the sonicated recombinant cell before induction; 2, 3, 4, 5 and 6 represent supernatant of sonicated recombinant cell induced for 1, 2, 3, 4 and 5 hours, respectively; 7 and 8 represent precipitate part of the sonicated cell debris induced for 4 and 5 hours, respectively. M is standard molecular weight of protein.
3. The inclusion bodies of fusion protein was purified by washing, and then dissolved in 8M urea solution for 1-2hr. Precipitate was removed by centrifugation. The solution was adjusted to pH 7.4-9.0, added nickel affinity gel for absorption for 1-2 hours. The resulted gel was placed into chromatography column, washed and gradient eluted with imidazole (20-500mM). The purified recombinant fusion proteins of apoptin-protein transduction domain of carboxyl-terminus of EC-SOD (Apoptin-EC-SOD-PTD) were collected.
4. The SDS-PAGE electrophoresis analysis of target protein is shown in FIGURE 7, wherein 1 represents sample solution before column chromatography, 2 represents sample collected in washing step, 3, 4, 5 and 6 respectively represent imidazole (20mM) elution fractions in turn, M is standard molecular weight of the protein, 7, 8 and 9 respectively represent imidazole (100mM) elution fractions in turn, 10, 11 and 12 respectively represent imidazole (500mM) elution fractions in turn.

### Example 5. Effects of fusion proteins of apoptin-protein transduction domain of carboxyl-terminus of EC-SOD on apoptosis of tumor cells cultured in vitro

1. The HeLa cells were cultured on the 96-well cell culture plate at 37°C until the cell density reached to about 60%. Imidazole was removed from the recombinant Apoptin-EC-SOD-PTD sample isolated and purified after expressing by repeated dialysis. After sterile filtration, the protein was diluted by adding different volume of DMEM cell culture media. From the highest concentration of protein, the concentration was reduced by half dilution gradually until zero. And the solution without protein was used as the negative control; for each protein concentration group were selected 3-4 wells for parallel control. Meanwhile, the recombinant Apoptin protein isolated and purified from the prokaryotic expression system using the similar method was used as the control and conducted the same experimental operation. The two sets of samples were cultured for 24h, drew culture media out and added the DMEM (100µl) containing the 5µg/ml of MTT, and then incubated for 4 hours at 37°C, drew the culture media out and added DMSO (100µl) to dissolve the purple crystals, incubated for 10min at 37°C and measured its absorbance at 490nm by a microplate reader. When the concentration of recombinant Apoptin- EC-SOD-PTD was 100µg/ml, the survival ratio of HeLa cells was 47% (IC50=105µg/ml). While at the same condition the effect of recombinant Apoptin (100µg/ml) in the control group and buffer solution of the negative group on the cell growth of HeLa was not obvious.
2. Results of HeLa cells apoptosis induced by recombinant Apoptin-EC-SOD-PTD and recombinant Apoptin were shown in FIGURE 8. Survival ratios of HeLa cells treated with different sample concentration were detected by MTT method.

### Example 6. Effects of fusion proteins of apoptin-protein transduction domain of carboxyl-terminus of EC-SOD on normal animal cells cultured in vitro

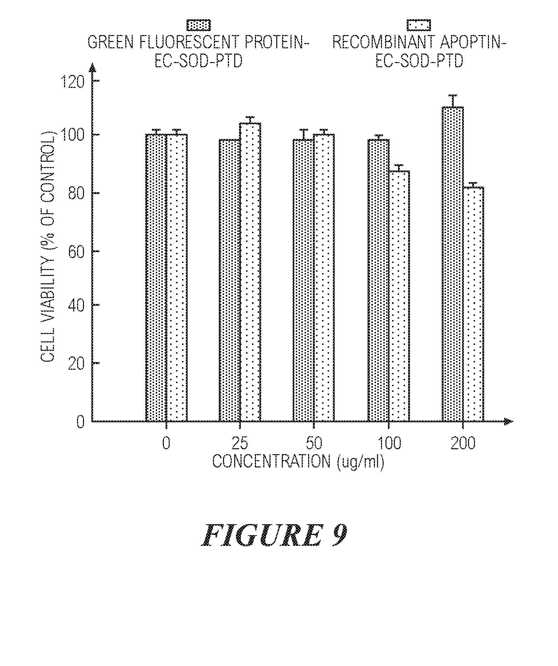

IC₅₀ of recombinant Apoptin-EC-SOD-PTD for normal human liver cell line L02 cultured *in vitro* was determined by MTT, and relevance between dose and cell toxicity thereof was observed.
1. The normal human liver cell line L02 was cultured in RPMI 1640 media containing 10% of fetal calf serum. After digested by pancreatic enzymes, the cultured cells were diluted with 1640 culture media to form a single cell suspension of 1×10⁵ cells per ml. 100µl dilution was inoculated into a 96-well cell culture plate. The inoculated cells were cultured in the incubator in presence of 5% CO₂ for 4h at 37°C.
2. The test drugs were diluted with culture media to a concentration of 200, 100, 50, 25, 0µg/ml. The volume of each well was 100µl, and each group has three parallel wells. The liver cells were continued to be cultured for 48h in incubator in presence of 5% CO₂ at 37°C.
3. MTT solution was added to each cultured well and cell survival ratio was determined by microplate reader colorimetric method. The experimental data were indicated by mean ± standard deviation.
4. The toxicity effect of the fusion proteins of apoptin-protein transduction domain of carboxyl-terminus of EC-SOD on normal animal cells cultured in vitro was shown in FIGURE 9. The recombinant Apoptin-EC-SOD-PTD has shown no significant effect on normal human hepatic cells cultured in vitro.

### Example 7. Antitumor effect of the fusion proteins of apoptin-protein transduction domain of carboxyl-terminus of EC-SOD on the animal model of mouse ascites tumor

The inhibition of tumor cells of the recombinant Apoptin-EC-SOD-PTD was testified through C57BL/6 mouse Lewis lung carcinoma model. Four groups including a blank control, a positive control and a recombinant Apoptin-EC-SOD-PTD test group and a recombinant Poly Arg-Apoptin group were set for the experiment, and 8 animals were tested for each group. After inoculation of Lewis lung carcinoma cells, the blank control group was injected with saline water. Whereas, the positive control group was injected with cyclophosphamide solution (20mg/kgBW/day). The two test groups were injected with the recombinant Apoptin-EC-SOD-PTD (10mg/kgBW/day) which was purified, dialysed and sterile filtrated and recombinant Poly Arg-Apoptin (40mg/kgBW/day), respectively.
1. Inoculation of carcinoma cell line: Six weeks old C57BL/6 male mice with a weight of about 20g were inoculated by armpit with 0.2ml of Lewis lung carcinoma single cell suspension. After feeding for 3-4 days, tumor growth in the armpits of the mice was observed. Drug administration test would be initiated when the tumor grew to a size of several millimeters.
2. Administration: Drugs were administered by intraperitoneal injection, and the administrations were continued for 7-8 days. The mouse was weighed before daily administration.
3. Detecting ratio of tumor inhibition: After the drug administrations were stop for 2 days, the mice were sacrificed by breaking their necks. The tumor tissue was stripped from the body, weighted and calculated the ratio of tumor inhibition. Ratio of tumor inhibition = (mean tumor weight of the blank group - mean tumor weight of the test group)/ mean tumor weight of the blank group x 100%.
4. The ratio of tumor inhibition of recombinant Apoptin-EC-SOD-PTD on the Lewis lung carcinoma was 37.8%, higher than that of the recombinant Ploy Arg-Apoptin, and close to that of the positive control group (the ratio of tumor inhibition of the positive control group was 47.4%). All these results indicated that the transmembrane effect of the apoptin significantly increased by linking to EC-SOD-PTD, which caused a significant growth inhibition effect to the tumor cells.

Antitumor effect of the fusion proteins of apoptin-protein transduction domain of carboxyl-terminus of EC-SOD on solid tumor

| | | | **Animal number** | | **weight of the tumor tissue** | |
|---|---|---|---|---|---|---|
| | | | beginning last | | **X±SD** | |
| Control group | -- | i.p | 8 | 7 | 2.3 ± 0.32 | -- |
| cyclophosphamide | 20 | i.p | 8 | 8 | 1.21± 0.119 | 47.4% |
| Poly Arg-Apoptin | 40 | i.p | 8 | 8 | 1.90±0.418 | 17.4 % |
| Apoptin-EC-SOD-PTD | 10 | i.p | 8 | 8 | 1.43±0.281 | 37.8% |

The scope of the present invention does not limited by the embodiments. The said embodiments are used as example to explain all aspects of the invention. The present invention includes functional equal methods and compositions. In fact, in addition to the content described herein, it is easy for the skilled person in the art to make various modification based on the description and the drawings. All these modifications fall into the scope of the claims of the present invention. And each reference mentioned above is incorporated in its entirety by reference.

## Claims

1. A fusion protein of apoptin-protein transduction domain of carboxyl-terminus of EC-SOD, which comprises protein transduction domain of carboxyl-terminus of EC-SOD in the amino acid sequence of SEQ ID NO: 1 or its mutants and apoptin in the amino acid sequence of SEQ ID NO:2 or its mutants.

2. The fusion protein of apoptin-protein transduction domain of carboxyl-terminus of EC-SOD according to claim 1, wherein the said fusion protein includes following formula: R2-R1, R1-R2, R1-L- R2-L-R1, R1-L-R2, R2-L-R1 or R2-L-R1-L-R1, in which R1 is a protein transduction domain of carboxyl-terminus of EC-SOD in the amino acid sequence of SEQ ID NO: 1 or its mutant, L is a linkage peptide, and R2 is an apoptin with at least one amino sequence of SEQ ID NO: 2 or its mutant.

3. The fusion protein of apoptin-protein transduction domain of carboxyl-terminus of EC-SOD according to claim 1, wherein the amino acid sequence of SEQ ID NO.1 of the said protein transduction domain of carboxyl-terminus of EC-SOD or its mutants is located at carboxy-terminus of the said fusion protein.

4. The fusion protein of apoptin-protein transduction domain of carboxyl-terminus of EC-SOD according to claim 1, wherein the said fusion proteins of apoptin-protein transduction domain of carboxyl-terminus of EC-SOD is protein represented by formula (a) or (b):
(a) Protein in the amino acid sequence of SEQ ID NO:3;
(b) Protein possessing at least 60% of amino acid sequences homology with that of (a), and capable to induce cell apoptosis.

5. A polynucleotide molecule encoding the said fusion protein of apoptin-protein transduction domain of carboxyl-terminus of EC-SOD according to any one of claims 1-4.

6. The polynucleotide molecule according to claim 5, which comprises the nucleic acid sequence of SEQ ID NO:4.

7. A recombinant expression vector comprising the said polynucleotide molecule according to claim 5.

8. The recombinant vector according to claim 7, wherein the said polynucleotide molecule in the nucleotide sequence of SEQ ID NO:4.

9. A transformant comprising the recombinant expression vector according to claim 7.

10. The transformant according to claim 9, wherein the said transformant is E. coli.

11. A preparation method for the fusion proteins of apoptin-protein transduction domain of carboxyl-terminus of EC-SOD according to any one of claim 1-4, which comprises following steps:
1. Constructing the expression vector for the said fusion proteins of apoptin-protein transduction domain of carboxyl-terminus of EC-SOD according to claim 1;
2. Preparing the transformant for the said fusion proteins of apoptin-protein transduction domain of carboxyl-terminus of EC-SOD according to claim 1;
3. Expressing the said fusion proteins of apoptin-protein transduction domain of carboxyl-terminus of EC-SOD according to claim 1;
4. Purifying the said fusion proteins of apoptin-protein transduction domain of carboxyl-terminus of EC-SOD according to claim 1.

12. The preparation method according to claim 11, wherein the transformant of the said fusion protein of apoptin-protein transduction domain of carboxyl-terminus of EC-SOD can be the prokaryotic or eukaryotic gene expression system.

13. A composition which includes the said fusion protein of apoptin-protein transduction domain of carboxyl-terminus of EC-SOD according to any of claims 1-4 and pharmaceutically acceptable carriers.

14. A use of the said fusion proteins of apoptin-protein transduction domain of carboxyl-terminus of EC-SOD according to any one of claims 1-4 for preparing drugs for treatment of disease caused by excessive cell proliferation.

15. The use according to claim 14, wherein the said disease caused by excessive cell proliferation is tumor.

16. The use according to claim 15, wherein the said tumor is selected from the group consisting of cervical cancer, breast cancer, liver cancer, or lung cancer.

17. A method for preventing, treating or improving disease or disorder caused by excessive cell proliferation, wherein the said method includes administering an effective amount of the fusion proteins of apoptin-protein transduction domain of carboxyl-terminus of EC-SOD according to claim 1, 2, 3 or 4 to a mammals in need.

18. The method according to claim 17, wherein the said disease caused by excessive cell proliferation is tumor.

19. The method according to claim 18, wherein the said tumor is selected from the group consisting of cervical cancer, breast cancer, liver cancer, or lung cancer.
